(19) Europäisches Patentamt

European Patent Office

Office européen des brevets

(11) Veröffentlichungsnummer: **0 490 250 A2**

# (12) EUROPÄISCHE PATENTANMELDUNG

(21) Anmeldenummer: **91120822.1**

(22) Anmeldetag: **04.12.91**

(51) Int. Cl.5: **A61K 37/02**, //(A61K37/02, 31:645,31:47,31:49,31:18,31:63)

(30) Priorität: **07.12.90 DE 4039114**

(43) Veröffentlichungstag der Anmeldung: **17.06.92 Patentblatt 92/25**

(84) Benannte Vertragsstaaten: **AT BE CH DE DK ES FR GB GR IT LI LU NL SE**

(71) Anmelder: **BOEHRINGER INGELHEIM INTERNATIONAL G.M.B.H.**

**W-6507 Ingelheim am Rhein(DE)**

(72) Erfinder: **Bienzle, Ulrich, Prof. Dr. Matterhornstrasse 5 W-1000 Berlin 37(DE)**

(54) **Verwendung von Interferon und eines antimalarisch wirkenden Wirkstoffs zur Behandlung von Malaria Infektionen.**

㊷ Die vorliegende Erfindung betrifft die Verwendung von Arzneimitteln auf der Basis von mindestens einem antimalarisch wirkenden Mittel, welche zusätzlich ein Interferon, vorzugsweise Interferon-gamma (IFN-γ), enthalten. Das erfindungsgemäße Arzneimittel findet Verwendung bei der Behandlung der klinischen Malaria.

EP 0 490 250 A2

Die vorliegende Erfindung betrifft die Verwendung von Interferon und einem antimalarisch wirkenden Wirkstoff zur Herstellung eines Arzneimittels für die Behandlung von Malaria-Infektionen in der erythrozytären (klinischen) Phase. Kennzeichnenderweise betrifft die Erfindung die Verwendung eines Arzneimittels auf der Basis von mindestens einem antimalarisch wirkenden Mittel, welches zusätzlich ein Interferon, vorzugsweise Interferon-gamma (IFN-γ), enthält. Die mit der erfindungsgemäßen Arzneimittelkombination zu behandelnden Malaria-Infektionen umfassen solche, die durch Protozoen der Gattung Plasmodium hervorgerufen werden, beispielsweise jene der Spezies Plasmodium falciparum, Plasmodium vivax, Plasmodium malariae, Plasmodium ovale, von denen bekannt ist, daß sie bevorzugt den Menschen infizieren, sowie Plasmodium berghei, Plasmodium knowlesi, Plasmodium vinckei, Plasmodium cynomologi, Plasmodium chabaudi, Plasmodium yoellii, die vor allem tierische Wirte, beispielsweise Nagetiere und Affen, befallen.

Obwohl die Malaria seit Jahrzehnten teils durch Vernichtung des Zwischenwirts mit Insektiziden teils durch therapeutische Maßnahmen bekämpft wird, stellt diese durch verschiedene Plasmodium Arten verursachte Infektionserkrankung bis heute weltweit eine der größten Gesundheitsprobleme dar. Schätzungsweise werden jährlich bis zu 200 Millionen in tropischen Ländern lebende Menschen mit Malaria-Erregern infiziert, mit letalen Folgen für 1 bis 2 Millionen davon betroffenen Menschen (STURCHLER, D., Experientia 40, 1357-1362, 1984).

Das bei der Malaria Bekämpfung bestehende Problem liegt hauptsächlich darin, daß beim Einsatz von Insektiziden immer wieder insektizid-resistente Zwischenwirte bzw. Vektoren (Anopheles) entstehen und die im Grunde nur prophylaktisch ausgerichtete medikamentöse Bekämpfung laufend Wirkstoff-resistente Protozoen (Plasmodium) entwickeln lassen (DOBERSTYN, E.B., Experientia 40, 1311-1317, 1984; BRUCE-CHWATT, L.J., Annu. Rev. Public Health 8, 75-110, 1987).

Seit der vor über 100 Jahren mit der Entdeckung des ursächlichen Erregers der Malaria eingeleiteten modernen Malaria Forschung (LAVERAN, A., Bull. Acad. Méd. Paris 9, 1235-1236, 1880) konnte kein Wirkstoff bereitgestellt werden, der eine Malaria-Infektion suffizient und nachhaltig therapierbar macht.

Durch den Stich des infizierten Moskitos werden Malariaparasiten (Sporozoiten) auf den Menschen übertragen. Diese Sporozoiten siedeln sich in der Leber an und vermehren sich dort. Nach Abschluß dieser Entwicklungsphase gelangen Parasiten (Merozoiten) in die Blutbahn und befallen Erythrozyten. Mit der Infektion der Erythrozyten beginnt die eigentliche klinische Krankheitsphase mit den bekannten Symptomen und Komplikationen.

Für die bisher nur prophylaktisch ausgerichtete Behandlung der Malaria bei Mensch und Tier wurden eine Anzahl an Maßnahmen vorgeschlagen, die die Verwendung animalarischer Wirkstoffe, die Immunisierung oder Vakzinierung und die Verwendung von Cytokinen umfassen.

Die bekannten animalarischen Wirkstoffe lassen sich hinsichtlich ihrer chemischen Konstitutionen in die folgenden 6 Hauptgruppen unterteilen:

1. die 9-Aminoacridine (z.B. Mepacrin),
2. die 4-Aminochinoline (z.B. Amodiaquin, Chloroquin, Hydroxychloroquin),
3. die 8-Aminochinoline (z.B. Primaquin, Quinocid),
4. die Biguanide mit Dihydrofolsäurereduktase inhibitorischer Wirkungsweise (z.B. Chlorproguanil, Cycloguanil, Proguanil),
5. die Diaminopyrimidine (z.B. Pyrimethamin),
6. die Quinin Salze.

Neben diesen Gruppen wurden auch Sulfone wie z.B. Dapson, Sulphonamide, Sulfanilamide und Antibiotika wie z.B. Tetracycline als antimalarische Mittel verwendet.

Je nach der Wirkungsweise können die bekannten antimalarischen Mittel in folgende Kategorien unterteilt werden:

1. Kausale prophylaktische Wirkstoffe, die gegen primäre Gewebestadien wirken,
2. gegen Rückfälle bzw. Rezidive gerichtete Wirkstoffe, die gegen latente Gewebestadien wirken,
3. Blut-Schizontozide,
4. Gametocytozide und
5. Sporontozide.

Zu der ersten Gruppe zählen beispielsweise Proguanil, Pyrimethamin und Primaquin und die Derivate davon und möglicherweise auch Sulfanilamide, Sulfonamide und Tetracycline. Für die zweite Gruppe stehen beispielsweise 8-Aminochinoline wie z.B. Primaquin und seine Analoge und Derivate zur Verfügung, sowie Floxacrin, Cycloguanil, Dapson, Quinazoline. Gegen die Blut-Schizonten, die dritte Kategorie, wirken insbesondere 4-Aminoacridine wie z.B. Mepacrin und die 4-Aminochinoline wie Chloroquin bzw. Chloroquinsulfat, Quinin, Amodiaquin und Mepacrin, Mefloquin und verwandte Verbindungen wie Halofantren, aber auch Pyrimethamin, Proguanil, Primaquin und die Sulfanilamide und Sulfonamide, insbesondere in Kombination mit Pyrimethamin.

Ferner kommen in Betracht die auf der Verbindung Artemisinin basierenden Sesquiterpenlactone und die semi-synthetischen Derivate davon, wie z.B. Artesunat und Artemether, sowie Piperaquin, Hydroxypiperaquin, Pyronaridin, Halofantren, wie auch generell die Biguanide und Quinin Salze.

Gegen die Gametocyten von beispielsweise P. vivax, P. malariae oder P. ovale sind auch die erwähnten Schizontozide wirksam, nicht jedoch gegen die maturen Gametocyten. Wirksam gegen die Gametocyten sind auch die 8-Aminochinoline wie z.B. Primaquin und Quinocid. Als sporontozid wirkende Mittel können Proguanil, Primaquin und Pyrimethamin genannt werden.Weiterhin sind als antimalarische Mittel bekannt: Chlorproguanil, Cycloguanil (z.B. als Salz der Embonsäure), Pamaquin, Plasmocid, Totaquin, Spirogermanium, Febrifugin, Brusatol, Brucein-A, Brucein-B, Brucein-C, Yadanziolid-A, Tebuquin, Enpirolin, Eurycomanon, 3-(4-Imidazolyl)-2-(pivaloylamido)propionylhydrazid, Cinchonidin, Cucurbitacine, Tripynadin, 5-Ethylthioribose, Arteether (Ethyläther Analogon von Artemether), Artenilsäure, Pyrexol, Atalaphillinin, Diformyldapson, Bruceantin, Nitroquin, Octanoylprimaquin, Pyrimethamin plus Sulfadoxin, Hivernin, Dabequin, Artelinsäure, Mefloquinquinat, Halofantrin-beta-glycerophosphat, Nimbolid, Sergeolid (Quassinoid von Picrolemma pseudocoeffea), Simalikalacton-D, Fluoroquin, Fluorenmethanol, Isouramil, Cycloleucin, Acedapson (Diacetyldapson), Gentiopicrin, Amquinat (Amquinolat), Endochin, Pentaquin, Isopentaquin, Methylchloroquin, Amopyroquin, Chinin, Hydrochinin (Dihydrochinin), Dimeplasmin, Azacrin, Diapromin, Menocton, Cycloquin (Haloquin), Lapinon, Aristoquin, Cloguanamil, Clociguanil, Brindoxim, Cinchonin, Tripiperaquin, 3-Hydroxy-2-(4-phenyl)cyclohexyl)-1,4-anthrachinon, Aminodiaquin, 4-Methyl-5-n-pentoxyprimaquin, 4-Methyl-5-n-hexoxyprimaquin, 2-(4-(4-Chlorphenyl) cyclohexyl)-3-hydroxy-1,4-naphtalindion, Gossypol-Derivate, Halofantrin (1,3-Dichlor-α-(2-(dibutylamino)ethyl)-6-(trifluormethyl)-9-phentantrenmethanol), Cinchona Alkaloide (z.B. in der Kombination Quinin, Quinidin, Cinchonin), N,N'-bis(3-((phenylmethyl) amino)propyl)-1,8-Octandiamin, N,N-bis (3-((phenyl-methyl)amino)-propyl)-1,7-diaminoheptan, Selenium-Analoge von 2-acetyl und 2-propionyl-pyridinthiosemicarbazone, Tebuquin, 2,6-bis (1-piperidinylmethyl)-4-((7-(trifluormethyl)-4-chinolinyl)amino)-phenol, primärer Phosphosäureester von 4'-Chlor-5-(1,1-dimethylethyl)-3-(((1,1-dimethyl ethyl)amino)methyl)-(1,1'-Biphenyl-2-ol, N4-(2,6-dimethoxy-4-methyl-5-(3-trifluoromethyl) phenoxy-8-chinolinyl)-1,4-pentandiamin, N,N-diethyl-N'-(6-methoxy-4-methyl-8-chinolinyl)-1,6-hexandiamin, 5-(N-aryl-tropan-3-yl)- und 5-(piperidin-4-yl)-2,4-diamino-pyrimidine, 4'-Amino-4-n-propylamino-2-methyldiphenylsulfon, 5-Ethylthioribose, Riboflavin-Analoge, 1-(3-(2,4-dichlorphenoxy)-1,6-diyhdro-6,6-dimethyl-1,3,5-triazin-2,4-diamin als Monohydrobromid, 1,6-dihydro-6,6-dimethyl-1-(3-(2,4,5-trichlorphenoxy)propoxy)-1,3,5-triazin-2,4-diamin als Monohydrochlorid, trans-2-(4-(1,1-dimethylethyl)cyclohexyl)-3-hydroxy-1,4-naphthalindion, Enpirolin, Mirincamycin, Tripynadin, 3-(4-Imidazolyl)- 2-(pivaloylamido)propionylhydrazid, 2-Acetylpyridin- thiosemicarbazone und seine Pyrrolidin-Derivate.

Die Verwendung dieser Stoffe allein oder in Kombination miteinander haben jedoch den Nachteil, daß nur eine vorbeugende oder nur vorübergehende Wirkung erzielt und eine mehr oder weniger rasche Resistenzbildung bei den jeweiligen Erregern ausgebildet wird, darüberhinaus wirken viele dieser Verbindungen toxisch oder entfalten nur in toxischen Konzentrationen ihre Wirkung (PETERS, W, Antimalarial drug resistance: an increasing problem, Br. med. Bull. 38, 187-192, 1982; YOUNG, M.D. and MORE, D.V. Chloroquine resistance in Plasmodium falciparum, Am. J. trop. Med. Hyg. 10, 317-320, 1961; BYGBJERG, I.C. et al., Mefloquin resistance of flaciparum malaria from Tanzania enhanced by treatment, Lancet 1, 21-26 1983; SCHMIDT, L.H., Antimalarial properties of floxacrine, a dihydroacridinedione derivative, Antimicrob. Agents Chemother 16, 475-485, 1979; BRUCE-CHWATT, L.J. Essential malariology, W. Heinemann Medical Books Ltd., London, 1980).

Viele dieser Verbindungen besitzen unerwünschte Nebenwirkungen oder können nur bei bestimmten Personengruppen verabreicht werden, auch verhindert eine zu kurze Plasma-Halbwertszeit mancher dieser Stoffe eine sinnvolle prophylaktische Verwendung (BRUCE-CHWATT et al., Chemotherapy of malaria, 2nd edn. WHO, Geneva 1981; COLBOURNE, M.J., Malaria Prophylaxis for long-term visitors, Comm. Dis. Rep. 35, 3-4, 1983; JIANG, J.B. et al., Antimalarial activity of mefloquine and quinghaosu, Lancet 2, 285-288, 1982.

Aufgrund der immer häufiger auftretenden Resistenzen gegen die genannten Verbindungen wurden verschiedene Kombinationen dieser Stoffe verwendet, beispielsweise Pyrimethamin mit Sulfadoxin (DOBERSTYN, E.B. et al., Single-dose therapy of falciparum malaria using pyrimethamine in combination with diformyldapsone or sulfadoxine, Am. J. trop. Med. Hyg. 25, 14-19, 1976; HALL, A.P. et al., Falciparum malaria cured by quinine followed by sulfadoxine pyrimethamin, Br. med. J. 2, 15-17, 1975; MERKLI, B. et al., The inhibitory effect of a drug combination on the development of mefloquine resistance in Plasmodium berghei, Ann. trop. Med. Parasit. 74, 1-9, 1980).

Jedoch lösten auch solche Kombinationen nicht das Problem der Resistenzentwicklung, außerdem ergaben sich Schwierigkeiten aufgrund von Nebenwirkungen (HURWITZ, E.S. et al., Resistance of Plasmodium falciparum malaria to sulfadoxine-pyrimethamine (Fansidar) in a refugee camp in Thailand, Lancet 1, 1068-70, 1981; PHILLIPS, R.E. et al., Failure of chloroquine-erythromycin and chloroquine-tetracycline combinations in treatment of chloroquinine resistent falciparum malaria in eastern Thailand, Lancet 1, 300-302, 1984; BJÖRKMAN A. & PHILLIPS - HOWARD, P.A., The epidemiology of drug resistant malaria, Trans. R. Soc. Trop. Med. Hyg. 84, 177-180, 1990).

Neben der Verwendung derartiger antimalarischer Wirkstoffe wurde vorgeschlagen, durch Immunisierung oder Vakzinierung eine Malariainfektion prophylaktisch zu behandeln.

Die Verwendung von verschiedenen Präparationen aus den unterschiedlichen Plasmodien-Stadien (Sporozoiten, Merozoiten, Schizonten, Gameten) zeigte jedoch unbefriedigende Ergebnisse, insbesondere aufgrund unerwünschter Autoimmunreaktionen und unspezifischer Immunantworten (TRAGER, W. et al., Immunisation of owl monkey to Plasmodium falciparum with merozoites from cultures of a knobless clone, Parasite Immun. 5, 255, 1983; WERNSDORFER, W.H., Prospect for the development of malaria vaccines, Bull, WHO 59, 335, 1981). Eine ideale Vakzine steht bisher nicht zur Verfügung (YOUNG, J.F., POSTE G., The prospects for a human malaria vaccine, TIBTECH 6, 63-68, 1988).

Neben den genannten Verfahren wurde die Verwendung von Interferon, z.B. Interferon-gamma (IFN-γ), sowohl zur Vakzinierung (PLAYFAIR, J.H.L., SOUZA, J.B., Recombinant gamma interferon is a patent adjuvant for a malaria vaccine in mice, Clin. exp. Immunol. 67, 5-10, 1987; HEATH, A.W. et al., Interferon-gamma as an adjuvant in immuno-compromised mice, Immunol. 67, 520-524, 1989), als auch zur therapeutischen Behandlung vorgeschlagen (BIENZLE, V. et al., Inhibition of Plasmodium vinckei - malaria in mice by recombinant murine interferon-γ, Acta Tropica 45, 289-290, 1988; MASHESHWARI, R.K. et al., Recombinant human gamma-interferon inhibits simian malaria, Infect. Immun. 53, 628-633, 1986; FERREIRA, A. et al., Inhibition of defelopment of exoerythrocytic forms of malaria parasites by γ-interferon, Science 232, 881-883, 1986; CLARK, I.A. et al., Inhibition of murine malaria (Plasmodium chabaudi) in vivo by recombinant interferon-γ or tumor necrosis factor, and its enhancement by butylated hydroxyanisole, J. Immunol 139, 3493-3496, 1987; SHEAR, H.L. et al., Role of IFN-γ in lethal and non lethal malaria in susceptible and resistant murine hosts, J. Immunol. 143, 2038-2044, 1989).

Im ersteren Fall wirkte das IFN-γ als Adjuvans in Verbindung mit einem entsprechenden Antigen, im zweiten Fall wurde zwar bei der prophylaktischen Verwendung eine Verlangsamung der Krankheitsentwicklung erzielt, die therapeutische Anwendung, d.h. post infectionem, war jedoch weniger effektiver und konnte die Parasitenvermehrung nicht suffizient inhibieren. Die kombinierte prophlyktische und therapeutische Anwendung von IFN-γ wirkte nur kumulativ. Ein Behandlungserfolg im Sinne einer kurativen Wirkung stellte sich in keinem Fall ein, da alle Tiere (Mäuse) nach allen drei Behandlungsweisen, gleichgültig wie lange und mit welchen Dosen die Tiere behandelt wurden, an der Malariainfektion starben (BIENZLE, V. et al., 1988, loc. cit.).

In dem US Patent 4,915,941 wird vorgeschlagen, IFNγ in Kombination mit einem antimalarisch wirkenden Mittel zur Prävention einer Malaria-Infektion einzusetzen, wobei die Gabe dieser Kombination nicht über die Präpatenzphase (Leberphase) hinausgeht. In dieser Phase ist eine Diagnose der Malariaerkrankung nicht möglich.

Überraschenderweise wurde nun gefunden, daß eine Kombination, bestehend aus Interferon, insbesondere IFN-γ plus mindestens ein antimalarisch wirkendes Mittel zur Behandlung der Malaria in der erythrozytären, d.h. klinischen, Phase geeignet ist, wobei erreicht wird, daß die Parasiten in den roten Blutzellen (Erythrozyten) vernichtet werden.

Zum prinzipiellen Unterschied gegenüber dem Stand der Technik wird es somit in unerwarteter Weise ermöglicht, die Behandlung der klinischen Malaria, d.h. einer nach den bekannten Verfahren diagnostizierbaren Malaria, nachhaltig durchzuführen.

Erfindungsgemäß wird somit die Verwendung von Interferon plus mindestens einem antimalarisch wirksamen Mittel zur Herstellung eines Arzneimittels für die Behandlung der erythrozytären, klinischen Malaria vorgeschlagen.

Überraschenderweise wurde auch gefunden, daß die kombinierte Verabreichung von Interferon mit mindestens einem antimalarisch wirkenden Mittel zu einer synergistischen Wirkungssteigerung bei der Behandlung der klinischen Malaria und zu einer anhaltenden, rezidivfreien Heilung dieser Erkrankung ohne Resistenzerscheinung bei nicht-immunen Individuen führte.

Es war ferner überraschend festzustellen, daß die Verabreichung des erfindungsgemäßen Arzneimittelkombination neben der synergistischen antimalarischen Wirkung eine zusätzliche, rasche Immunität gegenüber dem jeweiligen Erreger verleiht, und zwar schon nach einer einmaligen Infektion mit diesem Erreger.

Somit wird durch die Verwendung der erfindungsgemäßen Arzneimittelkombination zur Behandlung der klinischen Malaria ein mehrfacher Vorteil erreicht: Eine anhaltende kurative Behandlung der klinisch manifesten Infektion ohne Resistenzbildung bei nicht-immunen Individuen und eine rasche Ausbildung einer Immunität gegen den jeweiligen Erreger.

4

Im Gegensatz zur erfindungsgemäßen Verwendung der Arzneimittelkombination konnte bisher bei der Verabreichung der jeweiligen Monosubstanzen (antimalarisch wirkendes Mittel oder Interferon) meist nur eine geringe Verlängerung der Überlebenszeit gegenüber der mit Placebo behandelten Gruppe erreicht werden oder es wurde, wenn die Kombination IFN-γ plus antimalarisches Mittel beschrieben wurde, nur die Verwendung in der nicht-klinischen Präpatenzphase vorgeschlagen.

In einigen Fällen, wenn das post infectionem verabreichte antimalarisch wirkende Mittel, beispielsweise Chloroquin, hoch dosiert wurde, führte diese Behandlung zu einem Überleben einer Malaria Infektion; jedoch trat keine Schutzwirkung gegen eine Reinfektion mit demselben Erregerstammes ein.

Anhand von Reinfektionsexperimenten war bei der mit dem erfindungsgemäßen Arzneimittel behandelten Gruppe demgegenüber festzustellen, daß das Blut dieser Gruppe nicht infektös war und Schutz gegenüber einer Reinfektion durch den selben Erregerstamm bestand.

Als antimalarisch wirkende Mittel für das erfindungsgemäße Arzneimittel kommen die bekannten synthetischen, semi-synthetischen und natürlich vorkommenden Wirkstoffe in Betracht, wie sie beispielhaft in den vorgenannten Verbindungsgruppen, Verbindungen und Einzelwirkstoffen sowie in Form von Kombinationen davon vorliegen.

Die erfindungsgemäß zu verwendenden Interferone umfassen die natürlich vorkommenden, synthetisch und semi-synthetisch und die über DNA-Rekombination gentechnisch hergestellten Typ I- und Typ II-Interferone (Interferone α, β und γ), wobei das Interferon γ (IFN-γ) bevorzugt wird.

Das für die erfindungsgemäße Verwendung zu benutzende IFN-γ kann über die bekannten Methoden der konventionellen Zellkulturen tierischer bzw. menschlicher Herkunft hergestellt werden, beispielsweise gemäß W.R. BENJAMIN et al., Proc. Natl. Acad. Sci. USA. 79, 5379-5383, 1982; Y.K. YIP et al., Proc. Natl. Acad. Sci. USA 78, 1601-1605, 1981; Y.K.

Yip et al., Proc. Natl. Acad. Sci. USA 79, 1820-1824, 1982, oder über die ebenfalls bekannte Technik der DNA-Rekombination, beispielsweise gemäß P.W. GRAY et al., Nature 295, 503-508, 1982; E. RINDERKNECHT et al., J. Biol. Chem. 259, 6790-6797; R. DEVOS et al., Nucl. Acids Res. 10, 2487-2501, 1982; P.W. GRAY, D.V. GOEDDEL, Proc. Natl. Acad. Sci. USA 80 5842-5846, 1983 oder gemäß der EP-S 77 670, EP-A 271 824 oder nach TANIGUCHI et al., Nature 285, 547-549, 1980; GOEDDEL, D. et al., Nature 287, 411-416, 1980; EP-S 95 702, EP-A 280 033.

Bevorzugt wird für die erfindungsgemäße Verwendung das IFN-γ, insbesondere ein IFN-γ, welches durch DNA-Rekombination nach den bekannten Methoden erhalten werden kann.

Dem Durchschnittsfachmann ist bekannt, daß natürliche allele Variationen individuumspezifisch oder in verschiedenen Populationen vorkommen und sich durch eine oder mehrere unterschiedliche Aminosäuren oder durch unterschiedliche Nukleotide bzw. DNA-Sequenzen manifestieren. Derartige Variationen oder Mutationen, die auch durch die bekannten Methoden der DNA-Rekombination bzw. durch gezielte Mutagenese erzeugt werden können, wie sie beispielsweise von P.W. GRAY et al., 1982 loc. cit. und R. DEVOS et al., 1982, loc. cit. beschrieben werden, umfassen einfache oder mehrfache Substitutionen, Deletionen, Additionen, Insertionen oder Inversionen. Derartige IFN-γs werden daher erfindungsgemäß mitumfaßt.

Aus immunologischen Gründen ist dem Fachmann bekannt, daß er beim Einsatz von biologisch aktiven, körpereigenen Wirkstoffen vorzugsweise auf speziesspezifische Wirkstoffe zurückgreifen wird. Für die erfindungsgemäße, speziesspezifische Verwendung von Interferon wird demnach das aus dem jeweiligen, speziesspezifischen Geweben isolierte Interferon bevorzugt oder die aus den speziesspezifischen Geweben oder Zellen isolierten Nukleinsäuren (RNA, DNA) zur Herstellung des jeweiligen Interferons via DNA-Rekombination, insbesondere aber das dem jeweiligen, genuinem Interferon identische Polypeptid mit dem bekannten biologischen Aktivitätsspektrum von Interferon. Beispielsweise wird somit das für die erfindungsgemäße Verwendung am Menschen benutzte Interferon vorzugsweise ein IFN-γ sein, insbesondere ein human IFN-γ.

Für die erfindungsgemäße Anwendung von Interferon kommen die dem Fachmann bekannten und gebräuchlichen pharmazeutischen bzw. galenischen Formulierungen für die jeweilige Applikation in Betracht, vorzugsweise aber die für die parenterale Applikation, insbesondere für die intravenöse, intramuskuläre, subcutane, intracutane, intraartikuläre, intrathekale, intraperitoneale Infusion oder Injektion, wobei Dauerinfusionen oder intermittierende Infusionen mit den für den Fachmann zur Verfügung stehenden Pumpen oder die Verabreichung via mikroverkapselter Präparate z.B. auf Basis von Liposomen z.B. gemäß der EP-A 213 523 miteingeschlossen sind.

Zur Herstellung einer gebrauchsfertigen Lösung für die erfindungsgemäße Verwendung von Interferon stehen dem Fachmann die ihm zu diesem Zweck bekannten wässrigen Infusions- und Injektionslösungen zur Verfügung, gegebenenfalls zusammen mit den ihm bekannt Hilfs-, Träger - und/oder Stabilisierungsstoffen. Eine gebrauchsfertige Lösung für die erfindungemäße Verwendung ist beispielsweise auf die Weise herzustellen, in dem hochgereinigtes Interferon in "Wasser für Injektionszwecke" oder in mit Phosphat gepufferter physiologischer Salinelösung (pH 7 bis 7,5), gegebenenfalls mit Tween und/oder Gelatine oder einem Albumin supplementiert, vor der Applikation gelöst und in geeignete Gefäße (z.B. Spritzen, Ampullen, Beutel) steril abgefüllt wird.

Die zu verabreichende Menge an Interferon für die erfindungsgemäße Verwendung orientiert sich an den für den Fachmann bekannten Dosierungen, an der Schwere der Erkrankung, an der Ansprechrate und an dem weiteren Verlauf der Erkrankung sowie an den Nebenwirkungen. Allgemein ist daher davon auszugehen, daß die Dosierung nach individuellen Kriterien zu erfolgen hat.

Für die erfindungsgemäß zu verwendenden antimalarisch wirkenden synthetischen, semi-synthetischen oder natürlich vorkommenden Mittel, von denen mindestens eines davon in Kombination mit Interferon tierischen oder menschlichen Ursprungs vorliegt, kommen die bekannten und beschriebenen antimalarisch wirkenden Mittel in Betracht, vorteilhafterweise Verbindungen aus der Gruppe der 9-Aminoacridine, 4-Aminochinoline, 8-Aminochinoline, Biguanide, Diaminopyrimidine, Quinin Salze, Sulphonamide, Sulfanilamide, Antibiotika (wie z.B. Tetracycline) oder Sulfone (wie z.B. Dapson bzw. 4,4'-diaminodiphenylsulfon), beispielsweise Chloroquin [(7-chlor-4-(4-diethylamino-1-methylbutylamino)-chinolin]

Die Applikationsart und die Dosierung orientieren sich an den Therapieschemata, wie sie für die besagten antimalarischen Mittel bekannt sind, wobei auch solche auf Basis von z.B. Liposomen mikroverkapselte antimalarische Wirkstoffe z.B. gemäß der EP-A 213 523 oder der EP-A 152 379 wie auch z.B. gemäß der EP-A 354 442 oder der EP-S 56 781, um nur einige aus der Vielfältigkeit der publizierten Patentliteratur zu nennen, in Betracht kommen.

Die Verwendung des erfindungsgemäßen Arzneimittels auf der Basis von mindestens einem antimalarisch wirkenden Mittel plus einem Interferon kann entweder durch gleichzeitige Gabe der beiden verschiedenen Wirkstoffklassen (antimalarisch wirkendes Mittel, Interferon) als auch durch konsekutive oder sequenzielle Applikation über die jeweils geeignete Route erfolgen, wobei die einzelnen Wirkstoffe entweder räumlich und zeitlich getrennt, z.B. in Form eines "kit-of-part", oder unmittelbar räumlich und zeitlich zusammen vorliegen und appliziert werden können. Die getrennt oder unmittelbar oder mittelbar zusammen vorliegenden Wirkstoffkomponenten können sowohl als Trockensubstanzen als auch in Lösungen vorliegen, wobei auch mikroverkapselte Formen in Betracht kommen, bei denen die Wirkstoffkomponenten unmittelbar zusammen, mittelbar als Liposomenmischung oder als getrennte Verabreichungssysteme zur Anwendung kommen können. Vorteilhafterweise erfolgt die Applikation der beiden Wirkstoffkomponenten, antimalarisch wirkendes Mittel und Interferon, gleichzeitig.

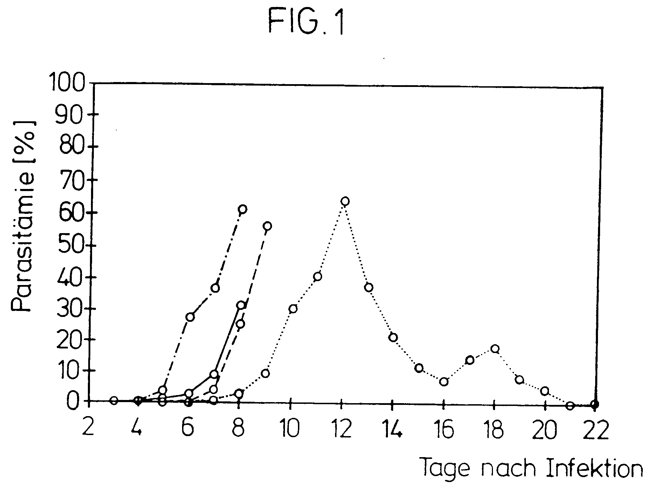

Legende zu Figur 1:

Verlauf einer Plasmodium vinckei Malaria in BALB/c Mäusen nach unterschiedlichen Behandlungen (n = 6 für jede Behandlungsgruppe) mit täglich einmal 80 $\mu$g Chloroquin (o o), 1 x $10^4$ U IFN$_\gamma$ (o----o), eine Kombination beider Wirkstoffe (o....o) oder als Kontrollgruppe phosphat gepufferte Saline (PBS) plus Mäusealbumin (o-.-.-o) 3 Tage vor bis 7 Tage nach der Infektion. Dargestellt sind die Mittelwerte der Parasitämien.

Beispiel

A. Infektion:

In allen Experimenten wurden weibliche, 8 bis 10 Wochen alte BALB/c Mäuse (Bundesgesundheitsamt Berlin) verwendet. Die Mäuse wurden mit P. vinckei durch intraperitoneale Injektion von $10^5$ parasitierten Erythrocyten, welche in 100 $\mu$l Phosphat gepufferte Saline (PBS) suspendiert waren, infiziert. Für die Reinfektionsexperimente wurden P. vinckei und P. berghei auf gleiche Weise verabfolgt. Drei Tage post infectionem wurden täglich dünne Blutausstriche durchgeführt und mit Giemsa Farbstoff angefärbt, um die Parasitämie zu bestimmen. Die statistische Analyse wurde mit dem Wilcoxon Test durchgeführt.

B. Behandlung mit Chloroquin:

Gruppen von je sechs mit P. vinckei infizierten Mäusen wurden am Tag der Infektion mit verschiedenen Dosen von Chloroquin (Bayer, Leverkusen) in Form einer einzigen intraperitonealen Applikation im Dosisbereich von 40 $\mu$g bis 300 $\mu$g pro Maus, verdünnt in 100 $\mu$l PBS, behandelt. Die Kontrolltiere erhielten nur PBS. Die unbehandelten Tiere (Kontrolltiere) erlagen der Infektion nach einer gemittelten Zeit von 8 Tagen. Bei den Mäusen, die mit einer hohen Dosis von 300 $\mu$g Chloroquin behandelt wurden, wurde nur eine niedrige und verzögerte Parasitämie beobachtet, wobei alle Mäuse überlebten. Demgegenüber überlebten nur zwei Mäuse der Gruppe, die 120 µg Chloroquin erhielten. Alle Mäuse, die mit 40 oder 80 µg Chloroquin behandelt wurden, starben innerhalb von 11 Tagen post infectionem. Eine signifikante (P<0.05) Verlängerung der Überlebenszeit im Vergleich zu den Kontrollen wurde bei den mit 80 µg Chloroquin behandelten Mäusen beobachtet, nicht jedoch bei jenen, die 40 µg Chloroquin erhielten. Daher wurde als subkurative Chloroquin Behandlung für die weiteren Untersuchungen 80 µg (4 mg/kg) gewählt.

C. Behandlung mit IFN-γ:

In dieser Gruppe (n = 6) wurden die Mäuse mit rekombinantem murinen IFN-γ aus E. coli (Genentech Inc. South San Francisco, Kalifornien, oder herstellbar nach P.W. GRAY, D.V. GOEDDEL, Proc. Natl. Acad. Sci. USA 80, 5842-5846, 1983) mit einer spezifischen Aktivität von 1.9 x $10^7$ U/mg Protein, gelöst in 0.1 % Mäuse Albumin enthaltendem PBS, behandelt. Die täglichen intraperitonealen Applikationen von je 100 µl mit entweder $10^4$ U IFN-γ pro Maus oder 5 x $10^4$ U IFN-γ pro Maus wurden über einen Zeitraum von 11 aufeinanderfolgenden Tagen gegeben, wobei die Behandlung 3 Tage vor der Infektion begonnen und 7 Tage nach der Infektion beendet wurde. Die Kontrolltiere erhielten PBS plus Mäuse Albumin (0.1 %). Die IFN-γ Gabe verzögerte signifikant (P<0.05) den Ausbruch einer eindeutigen Parasitämie und verlängerte die Überlebenszeit. Bei denjenigen Mäusen, die 1 x $10^4$ U IFN-γ/Tag über die Dauer von 11 Tagen verabreicht bekamen, verlängerte sich die Überlebenszeit im Vergleich zu den Kontrollen nur für 1 Tag, alle Mäuse starben.

Von den Mäusen jedoch, die 5 x $10^4$ U IFN-γ/Tag erhielten, überlebten drei von den sechs Mäusen die P. vinckei Malaria.

D. Behandlung mit IFN-γ plus Chloroquin:

Diese dritte Gruppe von Mäusen wurde mit 1 x $10^4$ U IFN-γ / Tag (wie unter C. beschrieben) in Kombination mit 80 µg Chloroquin (wie unter B. beschrieben) behandelt und sowohl mit dem Mäusen, die diese beiden Substanzen allein erhielten, oder mit der Kontrollgruppe, verglichen (Fig. 1).

Die Kontrollmäuse starben nach 9,0 Tagen (Mittelwert), die Chloroquin-behandelten Mäuse nach 10,25 Tagen (Mittelwert) und die IFN-γ behandelten Mäuse nach 10,0 Tagen (Mittelwert) post infectionem. Bei derjenigen Gruppe, die die kombinierte Behandlung erhielten, wurde die Parasitämie nur am Tag 5 oder am Tag 6 nach der Infektion offenkundig, im Vergleich zu Tag 3 oder Tag 4 in den anderen Gruppen. Bei den mit der Kombination behandelten Mäusen wurde der Peak der Parasitämie am Tag 12 erreicht.

Eine von sechs Mäusen mit der Kombinationsbehandlung starb am Tag 11. Bei drei der fünf überlebenden Mäuse zeigte sich ein zweiter kleinerer Peak einer Parasitämie, der ungefähr 18 Tage nach der Infektion in Erscheinung trat. Am Tag 22 hatten alle fünf Mäuse negative Befunde und blieben für mindestens fünf Wochen (bis zu den Reinfektionsexperimenten) parasitenfrei.

E. Reinfektionsexperimente:

Diejenigen Mäuse, die die P. vinckei Malaria aufgrund der unter D. beschriebenen kombinierten Behandlung mit IFN-γ und Chloroquin überlebten, wurden fünf bis zehn Wochen nach der ersten Infektion mit dem selben Stamm von P. vinckei reinfiziert.

Keine der Mäuse entwickelte eine erkennbare Parasitämie innerhalb eines Auswertungszeitraums von mehr als zehn Wochen. Mikroskopisch wurden jedoch Krisisformen gesehen. Das Blut aus diesen Mäusen wurde dann in ursprüngliche, nicht infizierte Mäuse injiziert, worauf sich keine Parasitämie entwickelte. Im Gegensatz dazu zeigten diejenigen Mäuse, die mit hohen Dosen Chloroquin geheilt wurden (wie unter B. beschrieben) keine Resistenz gegenüber einer Reinfektion mit dem selben Stamm.

Um zu bestimmen, ob diese bei der kombinierten Behandlung erreichte Immunität stammspezifisch ist, wurden die mit der Kombination behandelten überlebenden Mäuse entweder mit P. vinckei, P. berghei oder mit einer Kombination von P. vinckei plus P. berghei in dieser Reihenfolge infiziert und mit Kontrollmäusen verglichen, die mit den selben Parasiten infiziert wurden.

Hinsichtlich des Verlaufs der Parasitämie zeigten die immunen Mäuse, die mit P. berghei oder P. vinckei plus P. berghei infiziert waren, eine ähnliche Tendenz gegenüber einer Kontrollgruppe von mit P. berghei infizierten ursprünglichen BALB/c Mäusen. Die Gruppen ursprünglicher Mäuse, die entweder mit P. vinckei oder mit beiden Stämmen infiziert worden waren, zeigten einen früheren Anstieg der Parasitämie, verursacht durch die ungehinderte Multiplikation von P. vinckei (Tabelle 1).

Tabelle 1: Reinfektionsexperimente. Verlauf der gemittelten Parasitämien (Bereich) bei drei Gruppen von ursprünglichen Mäusen, die mit entweder Plasmodium berghei anka oder Plasmodium vinckei oder einer Kombination von P. berghei plus P. vinckei (jeweils n = 6) und bei drei Gruppen immuner Mäuse, die mit entweder P. berghei oder P. vinckei oder einer Kombination von P. berghei plus P. vinckei (jeweils n = 6) infiziert wurden.

| Tage nach der Infektion | 4 | 5 | 6 |
|---|---|---|---|
| mit P. berghei infizierte ursprüngliche Mäuse | 1 % (0.1-3) | 3.5 % (1-5) | 5.5 % (4-7) |
| mit P. berghei infizierte immune Mäuse | 0.5 % (0.1-1) | 3 % 2-6) | 6.5 % (5-11) |
| mit P. vinckei infizierte ursprüngliche Mäuse | 1 % (0.5-2) | 5 % (3-10) | 16 % (8-23) |
| mit P. vinckei infizierte immune Mäuse | 0 % (0-0) | 0 % (0-0) | 0 % (0-0) |
| mit P. berghei und P. vinckei infizierte ursprüngliche Mäuse | 3 % (3-4) | 9 % (7-10) | 12.5 % (11-14) |
| mit P. berhei und P. vinckei infizierte immune Mäuse | 0.5 % (0.1-2) | 4.5 % (2-7) | 8.5 % (5-11) |

**Patentansprüche**

1.  Verwendung von Interferon plus mindestens einem antimalarisch wirksamen Mittel zur Herstellung eines Arzneimittels für die Behandlung der erythrozytären, klinischen Malaria.

2.  Verwendung nach Anspruch 1, dadurch gekennzeichnet, daß das antimalarisch wirkende Mittel ein synthetischer, semi-synthetischer oder natürlich vorkommender Wirkstoff ist.

3.  Verwendung nach Ansprüchen 1 und 2, dadurch gekennzeichnet, daß ein darin enthaltenes antimalarisches Mittel ausgewählt ist aus den Gruppen der 9-Aminoacridine, 4-Aminochinoline, 8-Aminochinoline, Biguanide, Diaminopyrimidine, Quinin Salze, Sulfonamide, Sulfanilamide, Antibiotika und/oder Sulfone.

4.  Verwendung nach Ansprüchen 1 bis 3, dadurch gekennzeichnet, daß das darin enthaltene antimalarische Mittel ansgewählt ist aus der Gruppe der 4-Aminochinoline.

5.  Verwendung nach Anspruch 4, dadurch gekennzeichnet, daß das darin enthaltene antimalarische Mittel Chloroquin [(7-Chlor-4-(4-diethylamino-1-methylbutylamino)-chinolin] ist.

6.  Verwendung nach einem der Ansprüche 1 bis 5, dadurch gekennzeichnet, daß das darin enthaltene Interferon speziesspezifisch ist.

7.  Verwendung nach Anspruch 6, dadurch gekennzeichnet, daß das darin enthaltene Interferon ein aus natürlichen Zellen oder über DNA-Rekombination hergestelltes Interferon ist.

8.  Verwendung nach Anspruch 7, dadurch gekennzeichnet, daß das darin enthaltene Interferon IFN-gamma ist.

9.  Verwendung nach Ansprüchen 7 und 8, dadurch gekennzeichnet, daß das Interferon ein Human-Interferon ist.

10. Verwendung nach Ansprüchen 7 und 8, dadurch gekennzeichnet, das das Interferon ein tierisches Interferon ist.

# FIG.1